# FASCICULE DE BREVET EUROPEEN

(11) **EP 0 625 707 B1**
(45) Date de publication et mention de la délivrance du brevet: **01.09.1999**
(21) Numéro de dépôt: 94430003.7
(22) Date de dépôt: 18.05.1994
(51) Int. Cl.: G01N 33/50

(54) **Méthodes et composition pour la lyse des érythrocytes, composition de sang, et méthode d'analyse leucocytaire**
Verfahren und Zusammensetzung zum Lysieren von Erythrozyten, Blutzusammensetzungen und Verfahren zur Leukozytanalyse
Methods and composition for lysing erythrocytes, blood composition and method to analyse leucocytes

(30) Priorité: 19.05.1993 FR 9306333
(43) Date de publication de la demande: 23.11.1994
(73) Titulaire: Société Anonyme dite: IMMUNOTECH S.A., F-13276 Marseille Cédex 9 (FR)
(72) Inventeur: Van Agthoven, André, F-13009 Marseille (FR)
(74) Mandataire: Rinuy, Santarelli

(56) Documents cités:
- EP-A- 0 022 670
- EP-A- 0 161 770
- EP-A- 0 214 613
- EP-A- 0 530 490
- WO-A-85/05640

## Description

La présente invention concerne une méthode et une composition pour la lyse des érythrocytes, une composition de sang et une méthode d'analyse érythrocytaire.

Le sang contient plusieurs populations cellulaires. Les plus nombreuses sont les érythrocytes et les plaquettes fonctionnant dans l'échange et le transport de dioxyde de carbone et d'oxygène. Les plaquettes jouent un rôle dans la coagulation sanguine. Les leucocytes sont une population minoritaire impliquée dans le contrôle du système immunitaire.

Par analyse microscopique ou par analyse au cytomètre de flux, on distingue trois sous-populations dans les leucocytes : les cellules polynucléaires ayant plusieurs noyaux et les cellules mononucléaires avec un seul noyau, parmi lesquelles on peut distinguer les lymphocytes avec un noyau petit et rond et les monocytes avec un noyau de taille plus grande, réniforme.

Par cytométrie, en comparant la diffusion aux grands angles et la diffusion aux petits angles, on peut distinguer ces populations dans un cytogramme. La représentation de ces sous-populations leucocytaires est de 60 % environ pour les cellules polynucléaires, 30 % pour les lymphocytes et 10 % pour les monocytes. Des variations du pourcentage des sous-populations lymphocytaires peuvent donner une indication sur l'état de santé d'un individu.

Par marquage des cellules sanguines avec des anticorps monoclonaux conjugués à un marqueur de fluorescence et après analyse des cellules par microscopie, ou par cytométrie de flux, on peut distinguer les cellules sanguines en sous-populations encore plus finement qu'avec des moyens optiques simples.

Par exemple, avec le marqueur CD19, on peut distinguer dans la population des lymphocytes les cellules B qui sont dérivées de la moelle osseuse. Dans la même population, on peut distinguer, avec le marqueur CD3, les cellules T dérivées du thymus. Avec les marqueurs CD4 ET CD8, on peut distinguer respectivement les cellules T avec une fonction auxiliaire ou avec une fonction cytotoxique/suppresseur.
L'identification des sous-classes des lymphocytes est importante pour le diagnostic ou le traitement des maladies du système immunitaire.
Du fait que les érythrocytes sont une population majoritaire dans le sang, ils peuvent masquer les leucocytes et rendre difficile leur analyse par cytométrie de flux.

Les techniques d'immunofluorescence conventionnelle incluent une séparation physique des cellules lymphocytes et des érythrocytes, par exemple par centrifugation en gradient de densité (Boyum, A.1968 Scand.J.Clin. Lab Invest., 21 Suppl 97).

Une autre méthode, plus rapide, est la lyse des érythrocytes dans le sang total. Par exemple, dans la méthode de Hansen (US-A-4.284.412 - EP-A-0.022.670), un échantillon de sang traité avec un anti-coagulant est mélangé avec une préparation d'un anticorps conjugué fluorescent. Après incubation et lyse des érythrocytes, l'échantillon est passé dans un cytomètre de flux, afin d'analyser les populations leucocytaires positives pour l'anticorps en question.

Pour réaliser une analyse cytométrique correcte, il n'est pas seulement nécessaire de lyser tous les érythrocytes. Il faut aussi conserver tous les leucocytes dans un état morphologique où le cytomètre est capable de distinguer entre les cellules polynucléaires, les monocytes, les lymphocytes et le mélange de débris cellulaires et plaquettes. Il existe une grande diversité des méthodes de lyse érythrocytaire. Ces méthodes peuvent être basées, par exemple, sur un traitement acide, un traitement basique, un traitement à base de chlorure d'ammonium, d'alcool polyhydrique ou encore un choc hypotonique. Le problème de toutes ces méthodes est qu'en lysant les érythrocytes, des modifications sont introduites au niveau de la morphologie leucocytaire.

Dans les méthodes conventionnelles, on limite la dégradation leucocytaire, par exemple en procédant à l'analyse immédiatement après la lyse, ou en neutralisant après un choc hypotonique en acide, par lavage des cellules, ou par l'ajoût d'un réactif de fixation comme le formaldéhyde ou le paraformaldéhyde.

Les méthode de lyse utilisées par les fournisseurs de cytomètres sont adaptées aux caractéristiques optiques de cytomètres différents. Les cytomètres de Becton et Dickson Co. sont relativement insensibles aux changements de morphologie des leucocytes. Une lyse en conditions légèrement hypotoniques est indiquée pour ce type d'appareil (voir par exemple US-A-4.902.613).

Les cytomètres de marque Coulter sont beaucoup plus sensibles à une modification de morphologie leucocytaire. Un système de lyse Coulter (voir par exemple WO-89/0509) applique une lyse hypotonique et acide pendant environ 6 secondes et corrige ensuite la valeur isotonique et le pH du mélange. L'inconvénient d'un tel système est que la période de 6 secondes est difficilement réalisable dans une opération non-automatique.

Une autre méthode utilise le chlorure d'ammonium. Dans cette méthode, la valeur ionique de la solution est physiologique et la morphologie leucocytaire est bien conservée après une période courte. Le désavantage de cette méthode est que l'effet tensio-actif du chlorure d'ammonium entraîne une dégradation continue des leucocytes même après lavages des cellules et qu'on ne peut pas arrêter avec un réactif fixateur.

Il serait donc souhaitable de disposer d'une méthode d'analyse leucocytaire et de lyse érythrocytaire excluant tout lavage d'échantillons ainsi que la fausse numération des leucocytes.

C'est pourquoi la présente demande a pour objet une méthode et une composition pour lyser des cellules érythrocytaires d'un échantillon sanguin afin de ne pas former des agrégats importants avec les débris érythrocytaires pendant la lyse. La méthode de lyse selon la présente invention est assez efficace pour garantir une préparation d'échantillon stable avec une bonne séparation des sous-populations leucocytaires dans un cytogramme obtenu par cytométrie de flux. Les propriétés fluorescentes des sous-populations obtenues par marquage préalable avec des anticorps conjugués sont tout à fait conservées durant cette procédure.

La présente demande a donc pour objet une méthode de lyse des érythrocytes et de protection des leucocytes d'un échantillon sanguin, caractérisée en ce que l'on traite un échantillon de sang préalablement traité à l'aide d'un anti-coagulant, à l'aide d'une composition aqueuse de valeur ionique sensiblement physiologique comprenant un mélange
- d'un aldéhyde aliphatique,
- d'un alcool polyhydrique et,
- d'un sel d'un acide fort et d'un métal alcalin ou alcalino-terreux.

La composition ci-dessus est appelée dans ce qui suit: "composition de lyse".

L'aldéhyde aliphatique en quantité suffisante associé à l'alcool polyhydrique, conduit à une fixation de tous les composants cellulaires du sang et à une lyse des érythrocytes. Cette fixation est obtenue par un pontage entre les groupes amino terminaux des protéines dans la paroi cellulaire.
Par "aldéhyde aliphatique", l'on entend par exemple l'acétaldéhyde, le butyraldéhyde, le glyoxal et notamment le formaldéhyde et le paraformaldéhyde.

La concentration de l'aldéhyde aliphatique dans le mélange de sang et de composition de lyse va avantageusement de 0,14 à 0,72 M et de préférence de 0,36 à 0,50 M. Au-dessous de 0,10 M et au-dessus de 0,72 M la protection des leucocytes peut être suffisante mais on ne parvient pas à obtenir une lyse des érythrocytes.

Dans la présente demande, les quantités d'aldéhyde aliphatique, notamment du formaldéhyde, sont données pour une solution d'aldéhyde à 37 % P/V (13,3 M) stabilisé avec 10 % de méthanol (3,12 M) et sont donc des valeurs brutes d'aldéhyde.

La composition de valeur ionique sensiblement physiologique selon l'invention contient aussi un alcool polyhydrique, tel que l'éthylène glycol, le diéthylène glycol, le polyéthylène glycol et de préférence le glycérol.

La concentration finale de l'alcool polyhydrique dans le mélange de sang et de composition de lyse est avantageusement entre 0,44 et 2,2 M. La concentration préférée dans le mélange est entre 0,76 et 1,63 M. Au-dessous de la concentration de 3,2 %, la lyse des érythrocytes est insuffisante : au-dessus de 0,44 M, les débris érythrocytaires sont augmentés en volume et commencent à interférer avec la population des lymphocytes dans le cytogramme.

Pour obtenir une valeur ionique sensiblement physiologique, on utilise le sel précité d'acide fort, tel que d'acide chlorhydrique, sulfurique et de préférence perchlorique.

Le métal alcalin ou alcalino-terreux associé à l'acide fort est le lithium, le sodium, le potassium, le magnésium ou le calcium, et de préférence le sodium.
La concentration d'acide fort tel que de perchlorate dans le mélange sang- composition de lyse selon l'invention peut être, par exemple de 20 à 500 mM, et de préférence de 40 à 300 mM. L'ajustement de la valeur ionique à une valeur sensiblement physiologique, est de la compétence de l'homme de l'art.

De manière surprenante, alors qu'un aldéhyde aliphatique comme le formaldéhyde seul fixe les leucocytes et qu'un alcool polyhydrique comme le glycérol ne produit pas la lyse des érythrocytes, l'association de ces deux composés conduit à une lyse importante des érythrocytes.

Dans des conditions préférentielles de mise en oeuvre de la méthode ci-dessus décrite, la composition ci-dessus renferme en outre un alcool aliphatique notamment en C₁-C₆ tel que l'éthanol, le méthanol, et de préférence le propanol, le n-butanol ou l'isobutanol (2-méthyl propanol). La concentration de l'alcool aliphatique tel que l'isobutanol dans le mélange de sang et de réactif va avantageusement de 0,01 M à 0,54 M et, de préférence, de 0,05 M à 0,22 M. Au-dessous de 0,01 M, l'alcool aliphatique n'est pas efficace, au-dessus de 0,54 M, les débris érythrocytaires augmentent en volume de telle façon qu'ils interfèrent avec les lymphocytes dans le cytogramme.

Les alcools polyhydriques, les sels alcalins ou alcalino-terreux et les acides forts ont tous tendance à augmenter le volume des débris érythrocytaires.

Pour arriver à une conservation maximale de la morphologie leucocytaire, la valeur ionique de la composition ci-dessus doit prendre de préférence des valeurs physiologiques. On peut utiliser une valeur équivalente à environ 150 mM de chlorure de sodium, par exemple 150 mM ± 80 %, de préférence 150 mM ± 30 % et notamment 150 mM ± 10 %.

La composition ci-dessus renferme de préférence en outre une faible proportion d'au moins un acide faible. Celui-ci représente alors de préférence environ 10 à 100 mM du mélange sang et composition de lyse, et de préférence environ de 20 à 60 mM. L'acide faible peut être par exemple un des acides fumarique, malonique, succinique, pyruvique, lactique, aconitique, oxalique, acétique, formique, ascorbique, phosphorique, carbonique, tartrique et notamment citrique.

De par leur position dans le cytogramme, les monocytes sont particulièrement sensibles à la présence de métaux alcalino-terreux dans le réactif de lyse. La présence de ces composants améliore la diffusion aux petits angles des monocytes. Pour obtenir cette amélioration, la présence d'une faible quantité d'un métal et de préférence de deux métaux alcalino-terreux dans le mélange de sang et de réactif est préférée.

Par métaux alcalino-terreux, on entend par exemple le manganèse, le calcium et le magnésium. De préférence, on utilise un mélange de sels de magnésium et de calcium.

La concentration de magnésium peut aller d'environ 0,1 mM à 20 mM dans le mélange de sang et de réactif, et de préférence de 3 mM à 8 mM.

La concentration de calcium se situe notamment entre 0,1 et 20 mM dans le mélange et de préférence entre 3 mM et 8 mM. Comme contre-ion du magnésium et du calcium, on préfère un halogénure, de préférence un chlorure.

Le pH du mélange de sang et de réactif doit être situé entre 5 et 9, de préférence entre 6 et 8. Le pH est de préférence de 7 environ.

L'échantillon est mis en contact au moins 2 mn avec la composition ci-dessus, de préférence au moins 5 mn, et notamment environ 10 mn. On peut prolonger le contact jusqu'à plusieurs heures sans baisse notable de la qualité de l'analyse postérieure, ce qui montre l'excellente stabilisation leucocytaire du mélange.

La présente invention a également pour objet une composition aqueuse pour la lyse des érythrocytes et la protection des leucocytes, caractérisée en ce qu'elle comprend :
- un aldéhyde aliphatique,
- un alcool polyhydrique,
- un sel d'un acide fort et d'un métal alcalin ou alcalino-terreux
et en ce qu'elle a une valeur ionique sensiblement physiologique.

Rappelons qu'une valeur ionique physiologique est une valeur équivalente à environ 150 mM de chlorure de sodium.

Les compositions préférées sont celles définies ci-dessus dans le cadre des méthodes préférées.

On retient tout particulièrement la composition qui est une solution aqueuse à environ 0,54 M de formaldéhyde, environ 1,36 M de glycérol, environ 150 mM de perchlorate de sodium, environ 0,1 M d'isobutanol, environ 50 mM de citrate de sodium, environ 5 mM de chlorure de magnésium, environ 5 mM de chlorure de calcium et a un pH neutre de 7,0 environ.

Une méthode de numération des leucocytes d'un échantillon sanguin mettant en oeuvre la méthode de protection selon l'invention est notamment la suivante:
- Un échantillon de 0,1 ml de sang, traité avec un anti-coagulant est mis en contact avec une préparation d'un ou deux anticorps conjugués fluorescents, de préférence dans un volume de 20 µl. Après incubation, le mélange est mis en contact avec la composition selon l'invention. La composition est tout particulièrement une solution aqueuse à environ 0,54 M de formaldéhyde, environ 1,36 M de glycérol, environ 150 mM de perchlorate de sodium, environ 0,1 M d'isobutanol, environ 50 mM de citrate de magnésium, environ 5 mM de chlorure de magnésium, environ 6 mM de chlorure de calcium et a un pH neutre de 7,0 environ.
- Le volume de composition ci-dessus utilisé est par exemple de 0,2 ml à 1 ml pour 0,1 ml d'échantillon et de préférence d'environ 0,5 ml pour 0,1 ml d'échantillon.

Immédiatement après la mise en contact, le mélange est secoué et laissé à température ambiante pendant une période de 10 minutes. Ensuite, le volume total du mélange après lyse est, de préférence, augmenté par l'addition d'un volume de 0,5 ml d'une solution physiologique de chlorure de sodium (0,9 pour mille poids par volume - 154 mM) tamponnée par 8 mM de phosphate de sodium à pH 7,2 (PBS) afin d'augmenter le volume de l'échantillon en restant dans des conditions physiologiques. La lyse est considérée comme totale au bout de 10 minutes à l'issue desquelles l'échantillon est prêt pour l'analyse cytométrique.

Toute la procédure est effectuée de préférence à température ambiante (18-24°C).

Comme on l'a vu, la méthode de lyse de sang total selon l'invention a, selon les expériences de la demanderesse, une limitation vis-à-vis de l'appareil de cytométrie que l'on utilise. Les cytomètres de la Société COULTER^{R} tels EPICS PROFILE et EPICS EXCEL et ceux utilisant un système de lecture optique du même type, sont bien adaptés à une lyse de ce genre. Des cytomètres d'autres types peuvent donner un résultat décevant.

La présente demande a aussi pour objet une composition de sang dont les érythrocytes sont lysés et les leucocytes ont une morphologie substantiellement conservée, caractérisée en ce qu'elle comprend une mélange d'un échantillon de sang traité par un anticoagulant et d'une composition telle que définie ci-dessus.

La présente demande a aussi pour objet une méthode d'analyse de la population leucocytaire d'un échantillon sanguin dans laquelle l'on traite un échantillon sanguin à l'aide d'un anticoagulant, puis si désiré, à l'aide d'anticorps spécifiques d'au moins une population ou sous-population leucocytaire puis à l'aide d'un agent de lyse des érythrocytes, caractérisée en ce que après traitement à l'aide de l'anticoagulant et si désiré d'au moins un anticorps marqué, l'on traite l'échantillon à l'aide d'une composition aqueuse de valeur ionique sensiblement physiologique renfermant :
- un aldéhyde aliphatique,
- un alcool polyhydrique,
- un sel d'acide fort et d'un métal alcalin ou alcalino-terreux puis, de préférence, après dilution de l'échantillon à l'aide d'un diluant isotonique telle une solution physiologique (à 9 pour mille P/V) de chlorure de sodium tamponnée par 10 mM de phosphate de sodium à pH 7,2 (PBS), l'on procède à l'analyse cytométrique dudit échantillon.

La méthode ci-dessus peut notamment être mise en oeuvre dans les conditions préférentielles énoncées ci-dessus.

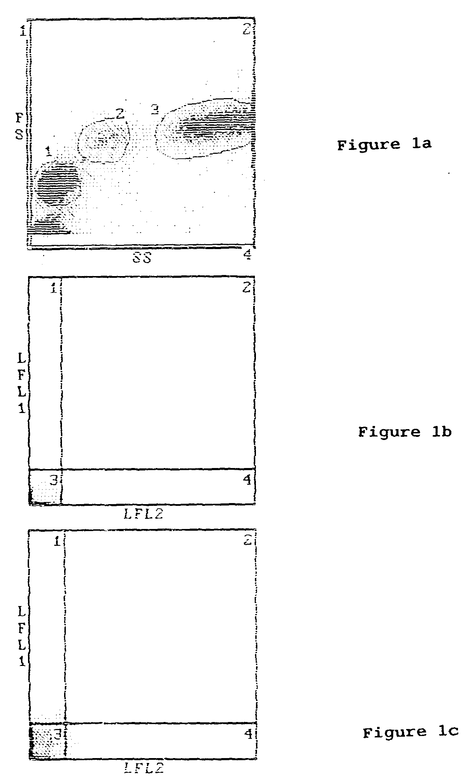

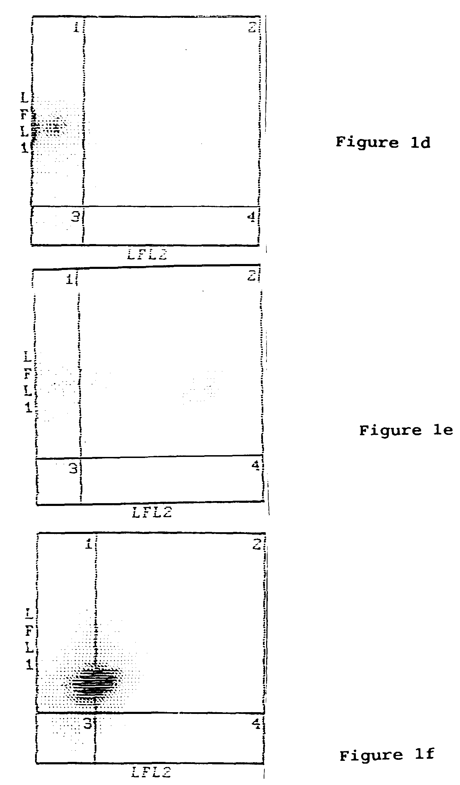

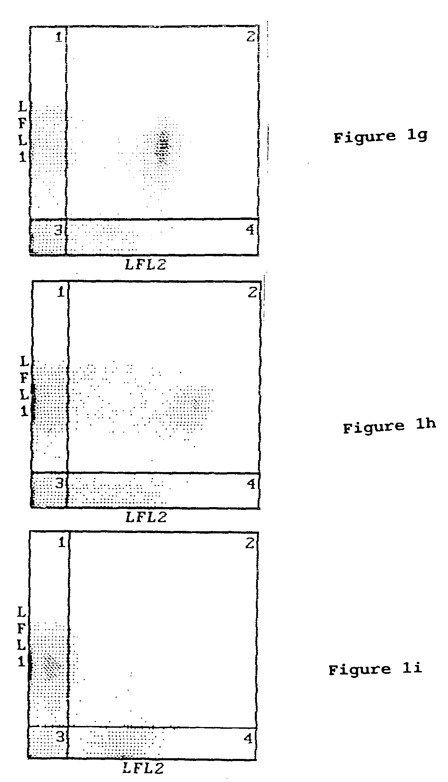

- La figure 1 montre un résultat de l'analyse de diffusion aux petits angles et de diffusion aux grands angles (fig.1a) ainsi que des diagrammes de double fluorescence (fig.1b à 1i) dans un cytogramme qui est obtenu en utilisant un cytomètre EXCEL EPICS PROFILE 1 ^{R}, d'un échantillon de sang total traité à l'aide de la composition de l'exemple 1.

Plus spécifiquement, la figure 1b représente un cytogramme de sang total en absence des marqueurs fluorescents.

La figure 1c représente un cytogramme de sang total en présence des marqueurs contrôles isotypiques. Diagramme sur le cadre R₁.

La figure 1d représente un cytogramme de sang total marqué avec CD45-FITC et CD14-PE. Diagramme sur le cadre R₁.

La figure le représente un cytogramme de sang total marqué avec CD45-FITC et CD14-PE. Cytogramme des cellules dans le cadre monocytaire (R₂).

La figure 1f représente un cytogramme de sang total marqué avec CD45-FITC et CD14-PE (Cytogramme des cellules dans le cadre granulocytaire (R₃).

La figure 1g représente un cytogramme de sang total marqué avec CD3-FITC et CD4-PE.

La figure 1h représente un cytogramme de sang total marqué avec CD3-FITC et CD8-PE.

La figure li représente un cytogramme de sang total marqué avec CD3-FITC et CD19-PE.

Les exemples qui suivent illustrent la présente invention sans toutefois la limiter.

### EXEMPLE 1 : Composition de lyse

On a préparé une composition aqueuse de lyse selon l'invention et ayant la composition suivante :
- formaldéhyde* 0,54 M
- glycérol 1,36 M
- perchlorate de sodium 0,150 M
- isobutanol 0,1 M
- chlorure de magnésium 0,005 M
- chlorure de calcium 0,005 M
- citrate de sodium 0,050 M
- eau distillée
pH = 7,0
* formaldéhyde stabilisé par 10 % de méthanol

### EXEMPLE 2 : Composition de lyse

On a préparé une composition de lyse aqueuse selon l'invention et ayant la composition suivante :
- formaldéhyde* 0,36 M
- glycérol 1,36 M
- perchlorate de sodium 0,150 M
- isobutanol 0,1 M
- fumarate de sodium 0,050 M
- eau distillée
pH = 7,0
* formaldéhyde stabilisé par 10 % de méthanol

### EXEMPLE 3 :

Des aliquots de 0,1 ml de sang traité par un anticoagulant sont répartis dans 6 tubes.

Dans le tube 1, on ajoute 20µl de PBS.

Dans le tube 2, on ajoute 20 µl d'un mélange de deux anticorps monoclonaux (contrôles isotypiques) avec une spécificité irrelevante, l'un conjugué à la fluorescéine isothiocyanate (FITC) référence 0639 (10 µl), l'autre à la phycoérythrine (PE) référence 0670 (2 µl), et de PBS (8 µl).

Dans le tube 3, on ajoute 20 µl d'un mélange d'anticorps monoclonal avec une spécificité CD45 conjugué à la FITC référence 0782 (2 µl), d'anticorps monoclonal avec une spécificité CD14 conjugué à la phycoérythrine réf. 0650 (2,5µl) et de PBS (15,5 µl).

Dans le tube 4, on ajoute 20 µl d'un mélange d'anticorps monoclonal avec une spécificité CD3 conjugué FITC référence 1281 (2 µl), d'anticorps avec une spécificité CD4 conjugué à la PE référence 0449 (10 µl) et de PBS (8 µl).

Dans le tube 5, on ajoute 20 µl d'un mélange d'anticorps CD3-FITC référence 1281 (2 µl), d'anticorps CD8-PE référence 0452 (3,3 µl) et de PBS (14,7 µl).

Dans le tube 6, on ajoute 20 µl d'un mélange d'anticorps CD3-FITC référence 1281 (2 µl), d'anticorps CD19-PE référence 1285 (10 µl) et de PBS (8 µl).

Les anticorps mentionnés ci-dessus sont commercialisés par la Société IMMUNOTECH, dans sa gamme IOTEST. Des anticorps comparables sont commercialisés par d'autre sociétés.

Après incubation de 20 minutes avec les anticorps, 0,5 ml de composition de l'exemple 1 est ajouté et les tubes sont immédiatement secoués. Après 10 minutes de réaction, un volume de 0,5 ml de PBS est rajouté et les tubes secoués. Après deux heures de conservation à température ambiante, les échantillons sont analysés en cytométrie de flux par un cytomètre EPICS PROFILE 1 de EXCEL. Dans l'analyse de diffusion aux petits angles et de diffusion aux grands angles (cytogramme), un seuil est placé afin d'éviter l'énumération de débris et de plaquettes. Un cadre est mis autour des populations de lymphocytes, monocytes et cellules polynucléaires. Une analyse en fluorescence 1 détectant les conjugués FITC et en fluorescence 2 détectant les conjugués PE, est effectuée sur les cellules lymphocytaires encadrées.

Les cellules positives en fluorescence pour CD45 représentent tous les leucocytes en permettant de distinguer entre les leucocytes et les débris plus plaquettes. Les cellules positives pour CD14 sont des monocytes permettant de distinguer entre monocytes et lymphocytes; les cellules positives pour CD3 et CD4 sont les cellules T auxiliaires. Les cellules positives pour CD3 et CD8 indiquent les cellules T cytotoxiques/suppresseur; les cellules positives pour CD3 sont les cellules T et les cellules positives pour CD19, les cellules B.
Les résultats sont représentés en figure 1.

L'analyse montre que dans le cadre lymphocytaire, 99 % des évènements sont de vrais lymphocytes, 70 % sont des cellules T, 17 % sont des cellules B et 12 % ne sont ni T, ni B.

Les 70 % de la population T, se répartissent en 49 % de cellules auxiliaires et 19 % de cellules cytotoxiques/suppresseurs.

Dans le cadre monocytaire, 93 % des événements sont de vrais monocytes.

Dans le cadre granulocytaire, 99 % des évènements sont de vrais granulocytes.

## Revendications

1. Méthode de lyse des érythrocytes et de protection des leucocytes d'un échantillon sanguin, caractérisée en ce que l'on traite un échantillon de sang préalablement traité à l'aide d'un anti-coagulant, à l'aide d'une composition aqueuse de valeur ionique sensiblement physiologique comprenant un mélange
- d'un aldéhyde aliphatique,
- d'un alcool polyhydrique et,
- d'un sel d'un acide fort et d'un métal alcalin ou alcalino-terreux.

2. Méthode selon la revendication 1, caractérisée en ce que l'échantillon est traité avec un anticorps spécifique d'au moins d'une sous-population lymphocytaire préalablement au traitement à l'aide de la composition aqueuse de valeur ionique sensiblement physiologique.

3. Méthode selon la revendication 1 ou 2, caractérisée en ce que la composition aqueuse de valeur sensiblement physiologique renferme en outre un alcool aliphatique.

4. Composition pour la lyse érythrocytaire, caractérisée en ce qu'il s'agit d'une composition aqueuse de valeur ionique sensiblement physiologique comprenant un mélange
- d'un aldéhyde aliphatique,
- d'un alcool polyhydrique et,
- d'un sel d'un acide fort et d'un métal alcalin ou alcalino-terreux.

5. Composition selon la revendication 4, caractérisée en ce que :
- l'aldéhyde aliphatique est le formaldéhyde
- l'alcool polyhydrique est le glycérol.

6. Composition selon la revendication 4 ou 5, caractérisée en ce que le sel d'un acide fort et d'un métal alcalin ou alcalino-terreux est un perchlorate.

7. Composition selon l'une quelconque des revendications 4 à 6, caractérisée en ce qu'elle renferme en outre un alcool aliphatique.

8. Composition selon l'une quelconque des revendications 4 à 7, caractérisée en ce qu'elle renferme une faible quantité d'au moins un métal alcalino-terreux choisi parmi le magnésium et le calcium.

9. Composition de sang traitée par un anticoagulant et lysée, caractérisée en ce qu'elle comprend un mélange d'un échantillon de sang traité par un anticoagulant et d'une composition aqueuse de valeur ionique sensiblement physiologique comprenant un mélange
- d'un aldéhyde aliphatique,
- d'un alcool polyhydrique et,
- d'un sel d'un acide fort et d'un métal alcalin ou alcalino-terreux.

10. Méthode d'analyse de population leucocytaire d'un échantillon sanguin dans laquelle l'on traite un échantillon sanguin à l'aide d'un anticoagulant, puis si désiré, à l'aide d'anticorps spécifiques d'au moins une population ou sous-population leucocytaire puis à l'aide d'un agent de lyse des érythrocytes, caractérisée en ce que après traitement à l'aide de l'anticoagulant et si désiré d'au moins un anticorps marqué, l'on traite l'échantillon à l'aide d'une composition aqueuse de valeur ionique sensiblement physiologique renfermant :
- un aldéhyde aliphatique,
- un alcool polyhydrique,
- un sel d'acide fort et d'un métal alcalin ou alcalino-terreux puis l'on procède à l'analyse cytométrique dudit échantillon.

## Patentansprüche

1. Verfahren zur Lyse von Erythrozyten und zum Schutz von Leukozyten einer Blutprobe, dadurch gekennzeichnet, dass man eine Blutprobe, die vorher mit einem Antikoagulans behandelt wurde, mit einer wässrigen Zusammensetzung mit im Wesentlichen physiologischer Ionenstärke, welche eine Mischung eines aliphatischen Aldehyds, eines Polyhydroxy-Alkohols und eines Salzes einer starken Säure und eines Alkali- oder Erdalkali-Metalls umfasst, behandelt wird.

2. Verfahren nach Anspruch 1, dadurch gekennzeichnet, dass die Probe vor der Behandlung mit der wässrigen Zusammensetzung mit im Wesentlichen physiologischer Ionenstärke mit einem Antikörper, der spezifisch gegenüber zumindest einer Lymphocyten-Subpopulation ist, behandelt wird.

3. Verfahren nach Anspruch 1 oder 2, dadurch gekennzeichnet, dass die wässrige Zusammensetzung mit im Wesentlichen physiologischem Wert weiters einen aliphatischen Alkohol umfasst.

4. Zusammensetzung für die Erythrozyten-Lyse, dadurch gekennzeichnet, dass es sich um eine wässrige Zusammensetzung mit im Wesentlichen physiologischer Ionenstärke, aufweisend eine Mischung eines aliphatischen Aldehyds, eines Polyhydroxy-Alkohols und eines Salzes einer starken Säure und eines Alkali- oder Erdalkali-Metalles, handelt.

5. Zusammensetzung nach Anspruch 4, dadurch gekennzeichnet, dass der aliphatische Aldehyd Formaldehyd ist, der Polyhydroxy-Alkohol Glycerol ist.

6. Zusammensetzung nach Anspruch 4 oder 5, dadurch gekennzeichnet, dass das Salz einer starken Säure und eines Alkali- oder Erdalkali-Metalls ein Perchlorat ist.

7. Zusammensetzung nach einem der Ansprüche 4 bis 6, dadurch gekennzeichnet, dass sie weiters einen aliphatischen Alkohol umfasst.

8. Zusammensetzung nach einem der Ansprüche 4 bis 7, dadurch gekennzeichnet, dass sie eine geringe Menge zumindest eines Erdalkali-Metalls, ausgewählt aus der Gruppe Magnesium und Calcium, umfasst.

9. Zusammensetzung von mit einem Antikoagulans behandeltem und lysiertem Blut, dadurch gekennzeichnet, dass sie eine Mischung einer mit einem Antikoagulans behandelten Blutprobe und einer wässrigen Zusammensetzung mit im Wesentlichen physiologischer Ionenstärke umfasst, welche eine Mischung eines aliphatischen Aldehyds, eines Polyhydroxy-Alkohols und eines Salzes einer starken Säure und eines Alkali- oder Erdalkali-Metalles umfasst.

10. Verfahren zur Analyse einer Leukozyten-Population einer Blutprobe, gemäß dem eine Blutprobe mit einem Antikoagulans, danach, wenn erwünscht, mit Antikörpern, die spezifisch gegenüber zumindest einer Leukozyten-Population oder -Subpopulation sind, danach mit einem Agens zur Erythrozyten-Lyse, behandelt wird, dadurch gekennzeichnet, dass nach der Behandlung mit dem Antikoagulans und, wenn erwünscht, zumindest einem markierten Antikörper, die Probe mit einer wässrigen Zusammensetzung mit im Wesentlichen physiologischer Ionenstärke, umfassend einen aliphatischen Aldehyd, einen Polyhydroxy-Alkohol, ein Salz einer starken Säure und eines Alkali- oder Erdalkali-Metalls, behandelt wird, und danach wird die zytometrische Analyse der Probe durchgeführt.

## Claims

1. Method of lysing erythrocytes and of protecting leukocytes of a blood sample characterized in that a sample of blood which has been pretreated with an anticoagulant, is treated with an aqueous composition having an ionic strength which is approximately physiological containing a mixture
- of an aliphatic aldehyde,
- of a polyhydric alcohol and,
- of a salt of a strong acid and an alkali metal or an alkaline earth metal.

2. Method according to claim 1, characterized in that the sample is treated with an antibody specific for at least one subpopulation of lymphocytes before the treatment with the aqueous preparation having an ionic strength which is approximately physiological.

3. Method according to claim 1 or 2, characterized in that the aqueous preparation having an ionic strength which is approximately physiological also contains an aliphatic alcohol.

4. A composition for lysing erythrocytes, characterized in that it is an aqueous preparation having an ionic strength which is approximately physiological containing a mixture
- of an aliphatic aldehyde,
- of a polyhydric alcohol and,
- of a salt of a strong acid and an alkali metal or an alkaline earth metal.

5. A composition according to claim 4, characterized in that:
- the aliphatic aldehyde is formaldehyde,
- the polyhydric alcohol is glycerol

6. A composition according to claim 4 or 5, characterized in that the salt of a strong acid and of an alkali metal or an alkaline earth metal is a perchlorate.

7. A composition according to any one of claims 4 to 6, characterized in that it also contains an aliphatic alcohol.

8. A composition according to any one of claims 4 to 7, characterized in that it contains a small quantity of at least one alkaline earth metal chosen form magnesium and calcium.

9. A composition of blood treated with an anticoagulant and lysed, characterized in that it contains a mixture of a sample of blood treated with an anticoagulant and with an aqueous composition having an ionic strength which is approximately physiological containing a mixture
- of an aliphatic aldehyde,
- of a polyhydric alcohol and,
- of a salt of a strong acid and an alkali metal or an alkaline earth metal.

10. A method of analyzing leukocyte populations of a blood sample in which a blood sample is treated with an anticoagulant, then, if desired, using at least one antibody specific for a leukocyte population or subpopulation, then using an erythrocyte lysing agent characterized in that after treatment using the anticoagulant and, if desired, using at least one labelled antibody, the sample is treated using an aqueous preparation having an ionic strength which is approximately physiological containing:
- an aliphatic aldehyde,
- a polyhydric alcohol and,
- a salt of a strong acid and an alkali metal or an alkaline earth metal then cytometric analysis of said sample is carried out.
